# EUROPÄISCHE PATENTSCHRIFT

(11) **EP 4 332 358 B1**
(45) Veröffentlichungstag und Bekanntmachung des Hinweises auf die Patenterteilung: **24.12.2025**
(21) Anmeldenummer: 22193477.1
(22) Anmeldetag: 01.09.2022
(51) Int. Cl.: F01P 11/14, G01N 17/02, G01N 33/28

(54) **VERFAHREN UND EINRICHTUNG ZUR BESTIMMUNG DES KORROSIONSSCHUTZVERMÖGENS EINER FLÜSSIGKEIT**
METHOD AND DEVICE FOR DETERMINING THE CORROSION PROTECTION CAPACITY OF A LIQUID
PROCÉDÉ ET DISPOSITIF DE DÉTERMINATION DE LA PUISSANCE D'UN LIQUIDE DE PROTECTION CONTRE LA CORROSION

(43) Veröffentlichungstag der Anmeldung: 06.03.2024
(73) Patentinhaber: Martechnic GmbH, 22459 Hamburg (DE)
(72) Erfinder: Matzke, Paul, 23552 Lübeck (DE); Lagner, Stefan, 22459 Hamburg (DE)
(74) Vertreter: Meissner Bolte Partnerschaft mbB

(56) Entgegenhaltungen:
- EP-A2- 0 224 323
- EP-A2- 0 412 746
- DE-A1- 102017 200 291

## Beschreibung

Die Erfindung betrifft ein Verfahren und eine Einrichtung zur Bestimmung des Korrosionsschutzvermögens einer Flüssigkeit nach dem Oberbegriff der Ansprüche 1 und 8.

Bei der Flüssigkeit handelt es sich insbesondere um Maschinenkühlmittel für Verbrennungskraftmaschinen, insbesondere von Schiffsmaschinen. Diese unterliegen bei ihrer Verwendung einer ständigen Alterung, so dass ihre Korrosionsschutzfähigkeit allmählich nachlässt. Kühlmittel dienen insbesondere der Kühlung der Maschinen und werden vor allem bei hohen Betriebstemperaturen stark belastet, so dass Inhaltsstoffe, insbesondere für den Korrosionsschutz, oxidieren können. Daher sind Kühlmittel regelmäßig auf ihr Korrosionsschutzvermögen zu überprüfen.

Eine derartige Überprüfung kann über aufwändige chemische oder physikalische Analysen erfolgen, indem diverse Einzelparameter darauf überprüft werden, ob das Korrosionsschutzvermögen weiterhin gegeben ist.

Es ist auch bekannt, das Korrosionsschutzvermögen über die Erfassung der elektrischen Leitfähigkeit zwischen zwei in die Kühlflüssigkeit eintauchenden Elektroden zu erfassen. In der DE 10 2015 204 717 A1 ist ein entsprechendes Verfahren angegeben. Dort wird in einer Probe oder unmittelbar im Kühlkreislauf einer Maschine zwischen den Elektroden ein festes Spannungspotential aufgebaut und mittels eines Amperemessgerät der Strom gemessen, der zwischen den Elektroden fließt. Die Auswertung erfolgt durch Vergleich des Wertes des fließenden Stroms in der zu messenden Kühlflüssigkeit mit dem Wert einer Neu-Kühlflüssigkeit bei gleichen Betriebsparametern.

Ein Problem bei derartigen Messungen besteht darin, dass auch die verwendeten Elektroden korrosionsanfällig sind und daher das Ergebnis einer Messung, insbesondere im Dauerbetrieb einer Messeinrichtung, unzuverlässig sein kann.

Aus der DE 10 2017 200291 A1 ist ein Verfahren zur Messung der Reinheit von Kühlwasser für eine Verbrennungsmaschine bekannt, bei dem mittels zweier Elektroden, die mit einem Gleichspannungspuls beaufschlagt werden, sich ein Energiespeicher in Abhängigkeit von der Leitfähigkeit des Wassers entlädt. Dabei wird ein Korrosionsschutzvermögen aus einer Zeitdauer der Entladung zwischen einem ersten und einem zweiten Ladezustand abgeleitet.

Aus der EP 0 224 323 A2 ist ein Probenbehälter bekannt, der zwei parallel angeordnete Elektroden aufweist. Die Einrichtung ist an eine Gleichspannungsquelle anschließbar und weist mehrere Energiespeicher auf.

Aus der EP 0 412 746 A2 ist ferner eine Vorrichtung zum Testen eines Fluids bekannt, wobei durch Messung der Leitfähigkeit des Fluids und dem Vergleich mit Referenzwerten die Qualität des Fluids bestimmt wird.

Der Erfindung liegt daher die Aufgabe zugrunde, ein Verfahren und eine Einrichtung zur Bestimmung des Korrosionsschutzvermögens einer Flüssigkeit, insbesondere einer Kühlflüssigkeit für Verbrennungskraftmaschinen anzugeben, welche eine verbesserte Genauigkeit der Messung, eine einfache Auswertemöglichkeit und die Verwendung einfacher Hardware erlaubt.

Diese Aufgabe wird durch die in den Ansprüchen 1 und 8 angegebene Erfindung gelöst. Weitergehende Ausgestaltungen der Erfindung sind in Unteransprüchen angegeben.

Bei der Erfindung werden zwei Elektroden in die auszuwertende Flüssigkeit eingebracht, wobei die Elektroden als eine korrosionsanfällige Arbeitselektrode und eine korrosionsfreie Gegenelektrode ausgebildet sind, wobei an die zwei Elektroden eine Gleichspannung angelegt ist, die aus einem Energiespeicher abgeleitet wird, der sich bei der Messung durch den Stromfluss zwischen den Elektroden entlädt. Als Qualitätskriterium für das Korrosionsschutzvermögen wird entweder die Zeitdauer zwischen einem ersten und einem zweiten Ladezustand des Energiespeichers oder dem Ladezustand des Energiespeichers nach Ablauf einer festgelegten Zeitdauer ab einem initialen Ladezustand ermittelt.

Vorzugsweise handelt es sich bei dem Energiespeicher um einen Elektrolytkondensator, der durch den Stromfluss zwischen den Elektroden langsam entladen wird und dessen Ladezustand durch Messung der Spannung an den Polen des Energiespeichers bestimmt wird. In einer bevorzugten Ausbildung des Verfahrens wird in einem ersten Schritt für eine festgelegte Zeitdauer ein konstantes Spannungspotential an die Elektroden angelegt, um die Elektroden zu initialisieren, das für eine korrosive Sensibilisierung der Arbeitselektrode sorgt. Anschließend werden die Elektroden in einem zweiten Schritt an den Energiespeicher angeschaltet, der sich dann durch den Stromfluss zwischen den Elektroden langsam entlädt. Die Entladespannung wird dabei kontinuierlich oder in festen Zeitabständen erfasst. Die Messung wird beendet, sobald eine festgelegte Zeitdauer nach der Anschaltung der Elektroden an den Energiespeicher erreicht ist oder die Entladespannung ein vorgewähltes Potential erreicht hat.

Die Einrichtung zur Bestimmung des Korrosionsschutzvermögens besteht aus einem Probenbehälter, in den zwei Elektroden parallel zueinander mit geringem Abstand in eine Probe der Kühlflüssigkeit eintauchen. Die Elektroden werden im ersten Schritt an ein konstantes Spannungspotential angeschlossen und im zweiten Schritt an einen Energiespeicher, der sich durch den Stromfluss über die Elektroden entlädt. Es ist eine Steuerschaltung vorhanden, die die Elektroden im zweiten Schritt von dem Spannungspotential des ersten Schritts auf das Spannungspotential des Energiespeichers des zweiten Schritts umschaltet.

Die Steuerschaltung ist auch dazu eingerichtet, im zweiten Schritt die Zeitdauer zwischen dem Ladezustand des Energiespeichers zu Beginn des zweiten Schritts und dem Erreichen eines gewählten Spannungspotentials bei der Entladung des Energiespeichers zu erfassen oder die Differenz zwischen dem Spannungspotential zu Beginn des zweiten Schritts und dem Spannungspotential zum Ablauf einer bestimmten Zeitdauer der Entladung zu erfassen.

Die Erfindung ermöglicht es, charakteristische Eigenschaften einer Verschlechterung des Korrosionsschutzvermögens eines Kühlmittels festzustellen, noch bevor die chemische Zusammensetzung und die physikalischen Eigenschaften Rückschlüsse darauf zulassen.

Die Erfindung wird nachstehend an Hand eines Ausführungsbeispiels näher erläutert. Es zeigen:

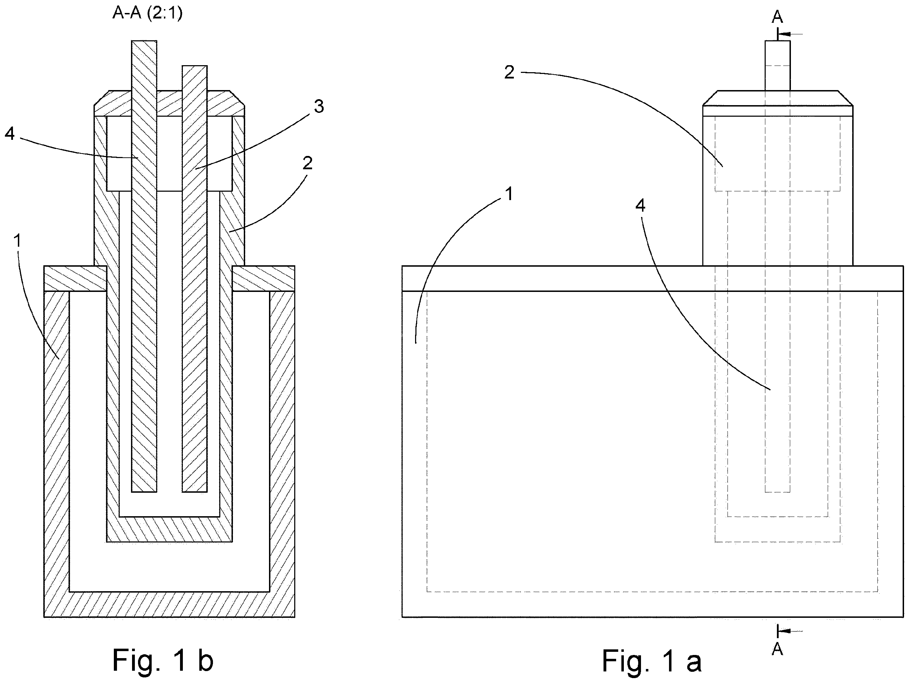

- Fig. 1 a: eine Seitenansicht eines Gehäuses mit einem Probenbehälter,
- Fig. 1 b: eine Schnittansicht eines Gehäuses mit Probenbehälter mit eingebrachten Elektroden,
- Fig. 2: eine Schaltungsanordnung zur Steuerung des erfindungsgemäßen Verfahrens, und
- Fig. 3: einen Programmablauf der Einrichtung zur Durchführung des Verfahrens.

Das in den Figuren 1 a und 1 b dargestellte Gehäuse weist eine Wandung 1 auf, die ein nicht gezeigtes Heizelement 9 enthält, das einen in das Gehäuse eingesetzten Probenbehälter umgibt. In einem Probenkopf 2 des Probenbehälters sind zwei stabförmige Elektroden 3 und 4 parallel zueinander mit kleinem Abstand verankert. Bereits benutzte Elektroden lassen sich aus dem Probenkopf entfernen und werden nach jeder Messung gegen ungebrauchte ausgetauscht.

Um die Wiederholbarkeit einer Messung sicherzustellen, werden definierte Ausgangsbedingungen in Hinblick auf Prüfvolumen, Probentemperatur, Prüfkörperoberfläche und Auslagerungsdauer hergestellt. Das Prüfvolumen beträgt vorzugsweise 30 - 50 ml, wobei für jede Messung das gleiche Prüfvolumen eingehalten sein sollte. Die Probentemperatur sollte im oberen Bereich des regelmäßigen Einsatzes des Kühlmittels liegen, welcher etwa 50 - 80 °C beträgt, wobei die Temperatur bei jeder Messung gleich sein sollte und vorzugsweise 60 °C beträgt. Die Einheitlichkeit der Prüfkörperoberfläche der Elektroden ergibt sich aus entsprechenden Vorgaben für die Fertigung. Die Arbeitselektrode, also diejenige Elektrode, die im Laufe der Messung korrosive Eigenschaften erhält und an den Minuspol der Prüfspannung angelegt wird, wird nach jeder Messung durch eine neue Elektrode ersetzt.

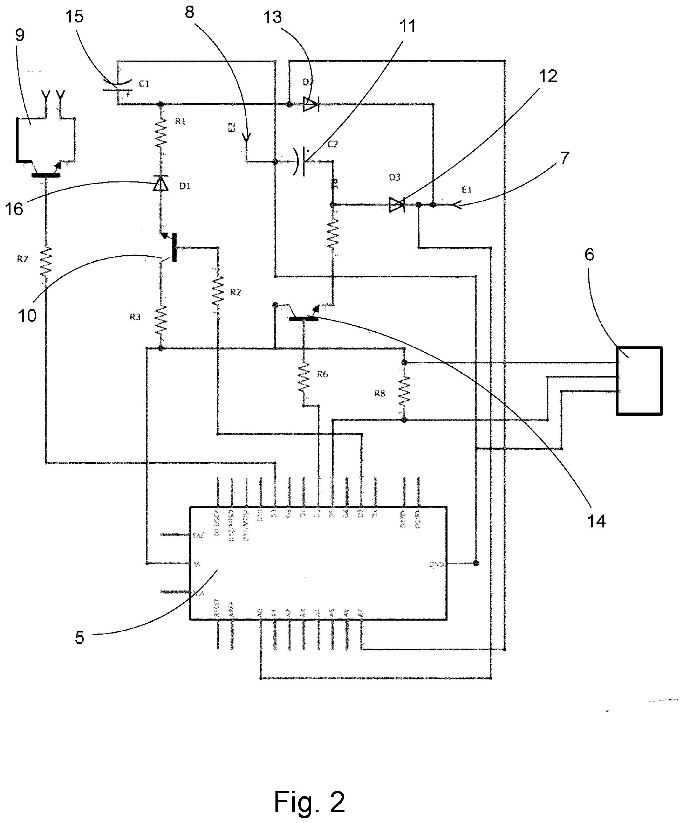

Figur 2 zeigt eine Steuerschaltung zur Durchführung des erfindungsgemäßen Verfahrens. Zentraler Bestandteil der Steuerschaltung ist ein Microcomputer 5, z.B. ein Arduino-Rechner. Dieser kann durch entsprechende Programmierung die erforderlichen Verfahrensschritte durchführen. Die Steuerschaltung ist vorzugsweise im Freiraum des Probenbehälters aufgenommen.

In der Vorbereitungsphase erfolgt das Einfüllen eines definierten Volumens der zu prüfenden Kühlflüssigkeit in den Probenbehälter. Dieser wird über das Heizelement 9 auf die Messtemperatur von 60 °C aufgeheizt. Die Aufheizung und Aufrechterhaltung der Messtemperatur wird über einen Temperaturfühler 6 von dem Microcomputer 5 gesteuert. Sobald die Messtemperatur erreicht ist, wird der Probenkopf 2 mit den Elektroden 3 und 4 auf den Probenbehälter aufgesetzt, so dass die Elektroden in definierter Länge in die Kühlflüssigkeit eintauchen.

Der folgende erste Schritt ist eine Reaktionsphase, in der eine aktive Polarisierung der Elektroden erfolgt. Hierzu wird an die Elektroden 3, 4 eine konstante Spannung von ca. 1 Volt angelegt, wobei die Arbeitselektrode an den Minuspol und die Gegenelektrode an den Pluspol der Spannungsversorgung angeschlossen wird. Während der Reaktionsphase (erster Schritt) sorgt das aufgebrachte Spannungspotential für eine korrosive Sensibilisierung der Arbeitselektrode. Durch die Freisetzung von Metallionen können dabei ebenfalls mögliche Inhibierungsmechanismen beschleunigt in Gang gesetzt werden. Die Dauer der Reaktionsphase orientiert sich an der für die Ausprägung eines Oberflächenschutzes der Arbeitselektrode notwendigen Zeit, nach der ein relativ stabiler Oberflächenschutz erreicht ist. Sie liegt bei einem Neu-Kühlmittel bei etwa 10 Minuten. Die Spannungshöhe liegt unterhalb der Spannung, die zur ungewünschten Elektrolyse führen könnte.

Die austauschbare Arbeitselektrode besteht vorzugsweise aus Kupfer, Grauguss, Messing oder einer Aluminiumlegierung und sollte dem zur Korrosion neigenden Material des Kühlkreislaufs, in dem das Kühlmittel zirkuliert, gleich oder ähnlich sein. Die Gegenelektrode ist vorzugsweise ein Edelmetall oder Edelstahl.

Die im ersten Schritt an die Elektroden über die Anschlüsse 7 und 8 angelegte Spannung von 1 Volt wird aus dem Microcomputer 5 über den Transistor 10 und die Diode 13 an den Anschluss 7 für die Gegenelektrode geleitet, wobei die Arbeitselektrode 8 an Masse liegt und die Diode 12 die Ladung des Kondensators 11 verhindert. Zur Stabilisierung der Spannung im ersten Schritt dient ein Kondensator 15 in der Größe von 20 pF.

Durch Abschalten des Transistors 10 wird die Phase des ersten Schritts beendet. Nun erfolgt die Ladung eines Elektrolytkondensators 11 über die Aufschaltung einer Ladespannung in Höhe von 5 Volt durch Einschalten des Transistors 14. Sobald die Ladespannung die vorgesehene Anfangsspannung des zweiten Schritts erreicht hat, wird die Aufladung des Kondensators 11 durch Abschaltung des Transistors 14 deaktiviert und der Kondensator 11 kann sich über die Elektroden 3 und 4, die an den Anschlüsse 7 und 8 angeschlossen sind, langsam entladen. Der Kondensator hat vorzugsweise eine Kapazität von 1 µF. Bei Wahl einer höheren Kapazität würde sich zwar die Entladezeit verlängern, aber die Gefahr bestehen, dass die Arbeitselektrode in den Bereich der Elektrolyse gelangt. Mit einem Wert von 1 µF beläuft sich die Messzeit auf etwa 20 - 100 Sekunden, wobei der untere Grenzwert der erfassten Spannung auf 0,5 Volt begrenzt ist. Die Dioden 16 und 13 isolieren den Ladestromkreis.

Der Microcomputer 5 erfasst durch interne Taktzählung die Zeitdauer, in der der die Spannung des Kondensators 11 von seinem Ausgangswert 5 Volt auf den Endwert 0,5 Volt abgefallen ist.

Die für ein beprobtes Kühlmittel erfasste Zeitdauer des Spannungsabfalls wird tabellarisch mit bereits früher ermittelten Werten bekannter Kühlmittel händisch ober über eine geeignete Programmierung des Microcomputers verglichen und an einer nicht dargestellten Anzeigeeinheit dargestellt. Da die Entladezeit des Kondensators 11 von dem Korrosionsschutzvermögen des Kühlmittels abhängt, korreliert eine längere Entladezeit mit einem besseren Korrosionsschutz der Arbeitselektrode.

Als Kondensator kommt vorzugsweise ein Elektrolytkondensator zum Einsatz. Für eine hohe Genauigkeit der Messung ist ein eng tolerierter Kondensator ≤ 5% von Vorteil. Ferner sollte die Spannungsmessung vorzugsweise mit einer Auflösung von 10 bit erfolgen, wobei ein hoher Eingangswiderstand von >100 MOhm gewählt werden sollte.

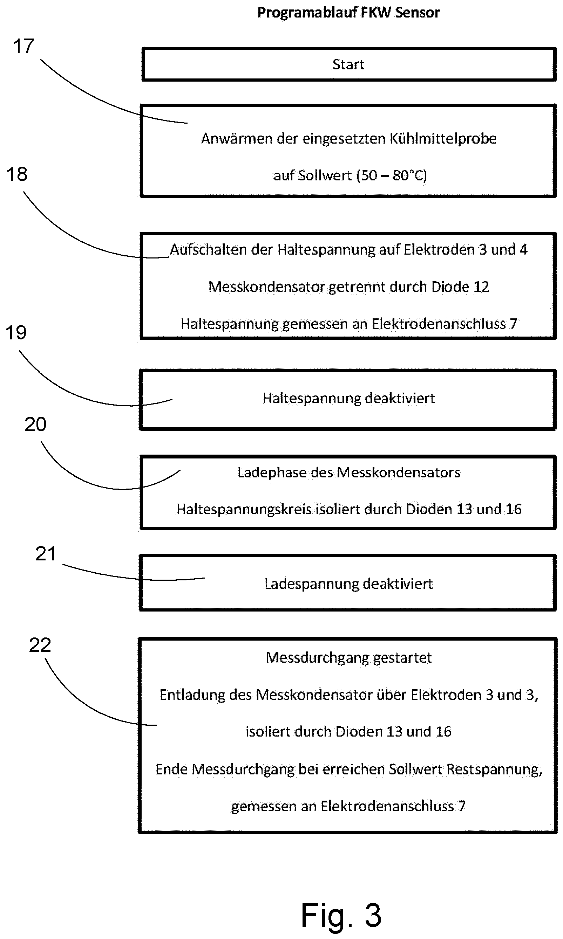

Figur 3 zeigt noch einmal den Ablauf des Verfahrens. Im Schritt 17 erfolgt das Anwärmen der Kühlmittelprobe auf Solltemperatur von 60 °C. In Schritt 18 wird die Haltespannung von 1 V aufgeschaltet, wobei die Diode 12 die Aufladung des Kondensators 11 noch verhindert. Nun wird in Schritt 19 durch Abschaltung des Transistors 10 die Haltespannung deaktiviert.

Es folgt Schritt 20, in dem der Kondensator 11 über den Transistor 14 auf eine Ausgangsspannung von 5 V geladen wird. Nach der Aufladung des Kondensators 11 wird für dessen Entladung der Transistor 14 abgeschaltet und in Schritt 22 der Messdurchgang gestartet. Die Entladung des Kondensators 11 erfolgt über die Anschlüsse 7 und 8 mit den daran angeschlossenen Elektroden. Sobald die Spannung am Kondensator 11 den Sollwert der Restspannung erreicht hat, wird der Messdurchgang beendet.

### Bezugszeichen

- 1: Wandung
- 2: Probenkopf
- 3: Elektrode
- 4: Elektrode
- 5: Microcomputer
- 6: Temperatursensor
- 7: Anschluss an Gegenelektrode
- 8: Anschluss an Arbeitselektrode
- 9: Heizelement
- 10: Transistor
- 11: Kondensator
- 12: Diode
- 13: Diode
- 14: Transistor
- 15: Kondensator
- 16: Diode
- 17: Anwärmen der Probe
- 18: Aufschalten Haltespannung
- 19: Haltespannung deaktiviert
- 20: Ladephase
- 21: Ladespannung deaktiviert
- 21: Entladung

## Patentansprüche

1. Verfahren zur Bestimmung des Korrosionsschutzvermögens einer Flüssigkeit, insbesondere einer Kühlflüssigkeit für eine Verbrennungsmaschine, mittels Feststellung der elektrischen Leitfähigkeit zwischen zwei mit Abstand zueinander in eine Probe der Flüssigkeit eingebrachte Elektroden (3, 4), wobei zwischen den Elektroden (3, 4) eine Gleichspannung angelegt ist, **dadurch gekennzeichnet, dass** die Elektroden (3, 4) als eine korrosionsanfällige Arbeitselektrode und eine korrosionsfreie Gegenelektrode ausgebildet sind, und wobei die Gleichspannung aus einem Energiespeicher (11) abgeleitet wird, der sich über die Elektroden (3, 4) in Abhängigkeit von der Leitfähigkeit der Flüssigkeit entlädt, und dass das Korrosionsschutzvermögen aus der Feststellung der Zeitdauer der Entladung zwischen einem ersten und einem zweiten Ladezustand des Energiespeichers oder dem Ladezustand des Energiespeichers nach Ablauf einer festgelegten Zeitspanne abgeleitet wird.

2. Verfahren nach Anspruch 1, **dadurch gekennzeichnet, dass** der Energiespeicher (11) ein Elektrolytkondensator ist.

3. Verfahren nach Anspruch 1, **dadurch gekennzeichnet, dass** der Ladezustand des Energiespeichers (11) durch Messung der Spannung an den Polen des Energiespeichers bestimmt wird.

4. Verfahren nach Anspruch 1, **dadurch gekennzeichnet, dass** in einem ersten Schritt für eine festgelegte Zeitdauer ein konstantes Spannungspotential an die Elektroden (3, 4) angelegt wird, und dass nach Ablauf der festgelegten Zeitdauer in einem zweiten Schritt eine selbständige Entladung des auf ein anfängliches Spannungspotential aufgeladenen Energiespeichers (11) erfolgt, wobei die Entladespannung des Energiespeichers kontinuierlich oder zu festgelegten Zeitpunkten gemessen wird.

5. Verfahren nach Anspruch 4, **dadurch gekennzeichnet, dass** die Elektroden (3, 4) im ersten Schritt an eine Spannungsquelle mit konstantem Spannungspotential angeschlossen sind und im zweiten Schritt an den aufgeladenen Energiespeicher (11) angeschaltet werden.

6. Verfahren nach Anspruch 4, **dadurch gekennzeichnet, dass** im ersten Schritt für eine Zeitdauer von 8 - 15 Minuten, vorzugsweise 10 Minuten, eine Spannung von 0,5 - 1,5 Volt, vorzugsweise 1 Volt, an die Elektroden (3, 4) angelegt wird und dass das anfängliche Spannungspotential 4,5 - 5,5 Volt, vorzugsweise 5 Volt, beträgt.

7. Verfahren nach einem oder mehreren der vorhergehenden Ansprüche, **dadurch gekennzeichnet, dass** die Bestimmung des Korrosionsvermögens der zu prüfenden Flüssigkeit durch tabellarischen Vergleich der Zeitdauer der Entladung des Energiespeichers oder der nach Ablauf einer festgelegten Zeitdauer bestimmten Entladespannung mit Vergleichswerten von in ihrer Qualität bekannten Proben einer Vergleichsflüssigkeit erfolgt.

8. Einrichtung zur Bestimmung des Korrosionsschutzvermögens einer Flüssigkeit zur Durchführung eines Verfahrens nach Anspruch 1, **dadurch gekennzeichnet, dass** ein Probenbehälter mit darin parallel zueinander angeordneten Elektroden (3, 4), welche als eine korrosionsanfällige Arbeitselektrode und eine korrosionsfreie Gegenelektrode ausgebildet sind, vorgesehen ist, dass eine Gleichspannungsquelle vorgesehen ist, wobei die Elektroden (3, 4) an die Gleichspannungsquelle anschließbar sind, welche dazu ausgebildet ist, in einem ersten Schritt ein konstantes Spannungspotential aufzuweisen, dass ein Energiespeicher (11) vorgesehen ist, welcher in einem zweiten Schritt an die Elektroden (3, 4) anschließbar ist und welcher dazu ausgebildet ist, sich über die in eine zu prüfende Flüssigkeit eingetauchten Elektroden (3, 4) zu entladen und dass eine Steuerschaltung (5) vorgesehen ist, die dazu ausgebildet ist, die Elektroden (3, 4) im zweiten Schritt von dem Spannungspotential der Gleichspannungsquelle mit konstanter Spannung auf das Spannungspotential des Energiespeichers (11) umzuschalten und wobei die Steuerschaltung (5) dazu eingerichtet ist, die Zeitdauer zwischen dem ersten Ladezustand am Beginn des zweiten Schritts und dem Erreichen eines zweiten Ladezustands zu erfassen oder das Spannungspotential des Energiespeichers nach Ablauf einer festgelegten Zeitdauer vom Beginn des zweiten Schritts festzustellen.

9. Einrichtung nach Anspruch 8, **dadurch gekennzeichnet, dass** der Energiespeicher (11) ein Elektrolytkondensator ist, der von der Gleichspannungsquelle mit konstanter Spannung aufgeladen wird, und dass die Steuerschaltung (5) dafür eingerichtet ist, im zweiten Schritt den Elektrolytkondensator zur Entladung über die Elektroden (3, 4) von der Gleichspannungsquelle mit konstanter Spannung zu trennen.

## Claims

1. A method for determining the corrosion protection capacity of a liquid, in particular of a coolant for an internal combustion engine, by means of establishing the electric conductivity between two electrodes (3, 4) introduced at a distance from one another into a sample of the liquid, wherein a DC voltage is applied between the electrodes (3, 4), **characterised in that** the electrodes (3, 4) are configured as a working electrode which is prone to corrosion and a corrosion-free counter electrode, and wherein the DC voltage is derived from an energy storage device (11) which discharges via the electrodes (3, 4) as a function of the conductivity of the liquid, and that the corrosion protection capacity is derived from the establishment of the length of time of the discharge between a first and a second state of charge of the energy storage device or the state of charge of the energy storage device after a specified period of time has elapsed.

2. The method according to Claim 1, **characterised in that** the energy storage device (11) is an electrolytic capacitor.

3. The method according to Claim 1, **characterised in that** the state of charge of the energy storage device (11) is determined by measuring the voltage at the poles of the energy storage device.

4. The method according to Claim 1, **characterised in that** a constant voltage potential is applied to the electrodes (3, 4) for a specified length of time in a first step, and that after the specified length of time has elapsed, the energy storage device (11) which is charged to an initial voltage potential discharges independently in a second step, wherein the discharge voltage of the energy storage device is measured continually or at specified times.

5. The method according to Claim 4, **characterised in that** the electrodes (3, 4) are connected to a voltage source with constant voltage potential in the first step, and are connected to the charged energy storage device (11) in the second step.

6. The method according to Claim 4, **characterised in that** a voltage of 0.5 - 1.5 volts, preferably 1 volt, is applied to the electrodes (3, 4) for a length of time of 8 - 15 minutes, preferably 10 minutes, in the first step, and that the initial voltage potential is 4.5 - 5.5 volts, preferably 5 volts.

7. The method according to one or more of the preceding claims, **characterised in that** the determination of the corrosion capacity of the liquid to be tested is carried out by tabular comparison of the length of time of the discharge of the energy storage device or of the discharge voltage determined after a specified length of time has elapsed with comparison values of samples of a comparison liquid known in terms of its quality.

8. A device for determining the corrosion protection capacity of a liquid for performing a method according to Claim 1, **characterised in that** a sample container is provided, which has electrodes (3, 4) arranged parallel to one another therein, said electrodes being configured as a working electrode which is prone to corrosion and a corrosion-free counter electrode, that a DC voltage source is provided, wherein the electrodes (3, 4) can be connected to the DC voltage source which is configured to have a constant voltage potential in a first step, that an energy storage device (11) is provided, which can be connected to the electrodes (3, 4) in a second step and which is configured to discharge via the electrodes (3, 4) immersed in a liquid to be tested, and that a control circuit (5) is provided, which is configured to switch over the electrodes (3, 4) from the voltage potential of the DC voltage source with constant voltage to the voltage potential of the energy storage device (11) in the second step, and wherein the control circuit (5) is designed to capture the length of time between the first state of charge at the start of the second step and the achievement of a second state of charge or to establish the voltage potential of the energy storage device after a specified length of time has elapsed from the start of the second step.

9. The device according to Claim 8, **characterised in that** the energy storage device (11) is an electrolytic capacitor which is charged by the DC voltage source with constant voltage, and that the control circuit (5) is designed to disconnect the electrolytic capacitor from the DC voltage source with constant voltage for discharging via the electrodes (3, 4) in the second step.

## Revendications

1. Procédé de détermination du pouvoir anticorrosif d'un liquide, en particulier d'un liquide de refroidissement pour un moteur à combustion interne, au moyen d'une détection de la conductibilité électrique entre deux électrodes (3, 4) introduites à une certaine distance l'une de l'autre dans un échantillon du liquide, une tension continue étant appliquée entre les électrodes (3,4), **caractérisé en ce que** les électrodes (3,4) sont réalisées comme une électrode de travail sensible à la corrosion et une contre-électrode exempte de corrosion et la tension continue étant issue d'un accumulateur d'énergie (11) qui se décharge par l'intermédiaire des électrodes (3, 4) en fonction de la conductibilité du liquide et **en ce que** le pouvoir anticorrosif est déduit de la détection de la durée de la décharge entre un premier et un second état de charge de l'accumulateur d'énergie ou de l'état de charge de l'accumulateur d'énergie après l'expiration d'un intervalle de temps défini.

2. Procédé selon la revendication 1, **caractérisé en ce que** l'accumulateur d'énergie (11) est un condensateur électrolytique.

3. Procédé selon la revendication 1, **caractérisé en ce que** l'état de charge de l'accumulateur d'énergie (11) est déterminé par la mesure de la tension aux pôles de l'accumulateur d'énergie.

4. Procédé selon la revendication 1, **caractérisé en ce que**, dans une première étape, un potentiel de tension constant est appliqué pour une durée définie aux électrodes (3, 4) et **en ce qu'**après l'expiration de la durée définie, dans une seconde étape, une décharge automatique de l'accumulateur d'énergie (11) chargé à un potentiel de tension initial s'effectue, la tension de décharge de l'accumulateur d'énergie étant mesurée en continu ou à des moments définis.

5. Procédé selon la revendication 4, **caractérisé en ce que**, dans la première étape, les électrodes (3, 4) sont raccordées à une source de tension présentant un potentiel de tension constant et, dans la seconde étape, elles sont branchées à l'accumulateur d'énergie (11) chargé.

6. Procédé selon la revendication 4, **caractérisé en ce que**, dans la première étape, on applique, pendant une durée de 8 - 15 minutes, de préférence de 10 minutes, une tension de 0,5 - 1,5 Volt, de préférence de 1 Volt, aux électrodes (3, 4) et **en ce que** le potentiel de tension initial est de 4,5 - 5,5 Volts, de préférence de 5 Volts.

7. Procédé selon l'une ou plusieurs des revendications précédentes, **caractérisé en ce que** la détermination du pouvoir corrosif du liquide à tester s'effectue par une comparaison sous forme de tableau de la durée de la décharge de l'accumulateur d'énergie ou de la tension de décharge déterminée après l'expiration d'une durée définie à des valeurs comparatives d'échantillons connus en ce qui concerne leur qualité d'un liquide comparatif.

8. Dispositif de détermination du pouvoir anticorrosif d'un liquide pour la mise en œuvre d'un procédé selon la revendication 1, **caractérisé en ce qu'**un récipient à échantillon présentant des électrodes (3, 4) agencées parallèlement l'une à l'autre, qui sont réalisées comme une électrode de travail sensible à la corrosion et une contre-électrode exempte de corrosion, est prévu, **en ce qu'**une source de tension continue est prévue, les électrodes (3, 4) pouvant être raccordées à la source de tension continue, qui est réalisée pour présenter, dans une première étape, un potentiel de tension constant, **en ce qu'**un accumulateur d'énergie (11) est prévu, qui peut être raccordé, dans une seconde étape, aux électrodes (3, 4) et qui est réalisé pour se décharger par l'intermédiaire des électrodes (3, 4) immergées dans un liquide à tester et **en ce qu'**un circuit de commande (5) est prévu, qui est réalisé pour commuter les électrodes (3, 4), dans la seconde étape, à partir du potentiel de tension de la source de tension continue présentant une tension constante au potentiel de tension de l'accumulateur d'énergie (11) et le circuit de commande (5) étant conçu pour enregistrer la durée entre le premier état de charge au début de la seconde étape et l'obtention d'un second état de charge ou pour détecter le potentiel de tension de l'accumulateur d'énergie après l'expiration d'une durée définie depuis le début de la seconde étape.

9. Dispositif selon la revendication 8, **caractérisé en ce que** l'accumulateur d'énergie (11) est un condensateur électrolytique qui est chargé par la source de tension continue présentant une tension constante et **en ce que** le circuit de commande (5) est conçu pour séparer, dans la seconde étape, le condensateur électrolytique pour la décharge par l'intermédiaire des électrodes (3,4) de la source de tension continue présentant une tension constante.
